# EUROPÄISCHE PATENTANMELDUNG

(11) **EP 4 641 579 A1**
(43) Veröffentlichungstag der Anmeldung: **29.10.2025**
(21) Anmeldenummer: 24172092.9
(22) Anmeldetag: 24.04.2024
(51) Int. Cl.: G16H 20/17, G16H 40/40, H04L 9/32, A61M 5/172

(54) **INFUSIONSPUMPENSYSTEM, VERFAHREN ZUR SICHEREN DATENÜBERTRAGUNG FÜR INFUSIONSPUMPENSYSTEM UND COMPUTERLESBARES SPEICHERMEDIUM**

(71) Anmelder: B. Braun Melsungen AG, 34212 Melsungen (DE)
(72) Erfinder: OBERMANN, Dennis, 34628 Willingshausen (DE); MOELLER, Sebastian, 37235 Hessisch Lichtenau (DE)
(74) Vertreter: Winter, Brandl - Partnerschaft mbB

(57) **Zusammenfassung**

Die vorliegende Offenbarung betrifft ein Infusionspumpensystem (20), das eine erste Infusionspumpe (2) und eine zweite Infusionspumpe (4) aufweist, die miteinander für eine sichere Datenübertragung gekoppelt sind/ koppelbar sind, wobei das Infusionspumpensystem (20) eingerichtet ist, dass zumindest eine aus der ersten und der zweiten Infusionspumpe (2, 4) mit einer digitalen Signatur (16) signierte Daten erhält, und/oder zumindest eine aus der ersten und der zweiten Infusionspumpe (2, 4) mit einer Verschlüsselung (22) verschlüsselte Daten erhält. Weiter betrifft die Offenbarung ein Verfahren zur sicheren Datenübertragung für ein Infusionspumpensystem (20) und ein computerlesbares Speichermedium (26).

## Beschreibung

Die Offenbarung betrifft ein Infusionspumpensystem, ein Verfahren zur sicheren Datenübertragung für ein Infusionspumpensystem und ein computerlesbares Speichermedium.

### Hintergrund der Offenbarung

In der automatisierten Infusionstechnik werden Infusionspumpen miteinander gekoppelt, um gemeinsam Therapien zu realisieren. Ein Beispiel dafür ist der sogenannte Take-Over-Mode. Dieser unterstützt den Anwender während des Spritzenwechsels einer Spritzenpumpe, indem er automatisch eine zweite Spritzenpumpe startet, sobald die erste Spritzenpumpe leergelaufen ist. Hierzu werden Informationen zwischen der Spritzenpumpe, die leerläuft, und der Spritzenpumpe, die anschließend die Behandlung übernimmt, ausgetauscht. Diese sind nötig, um Infusionsparameter zu synchronisieren oder Aktionen, wie das Starten der übernehmenden Spritzenpumpe, zu initiieren.

Bei aktuellen Take-Over-Mode-Implementierungen und (einigen) anderen Implementierungen zur Datenübertragung zwischen Infusionspumpen werden diese Informationen nicht (besonders) sicher übertragen. Es wäre also möglich, dass diese Informationen abgegriffen und manipuliert werden.

### Kurzbeschreibung der Offenbarung

In Anbetracht der oben beschriebenen Problematik ist es daher eine Aufgabe der Offenbarung, eine sichere Datenübertagung in einem Infusionspumpensystem mit zumindest zwei Infusionspumpen zu gewährleisten, oder zumindest die Datenübertragung sicherer zu machen.

Diese Aufgabe wird durch ein Infusionspumpensystem nach Anspruch 1, ein Verfahren zur sicheren Datenübertragung für ein Infusionspumpensystem nach Anspruch 11 und ein computerlesbares Speichermedium nach Anspruch 15 gelöst. Vorteilhafte Ausführungsformen sind in den abhängigen Ansprüchen beansprucht und/oder nachfolgend in der Beschreibung beschrieben.

Das offenbarungsgemäße Infusionspumpensystem weist eine erste Infusionspumpe und eine zweite Infusionspumpe auf, die miteinander für eine sichere Datenübertragung gekoppelt sind/ koppelbar sind, wobei das Infusionspumpensystem eingerichtet ist, dass zumindest eine aus der ersten und der zweiten Infusionspumpe mit einer digitalen Signatur signierte Daten erhält, und/oder zumindest eine aus der ersten und der zweiten Infusionspumpe mit einer Verschlüsselung verschlüsselte Daten erhält.

Unter verschlüsselten Daten bzw. verschlüsselt ist also zu verstehen, dass die Daten mit einer entsprechenden Verschlüsselung verschlüsselt sind. Unter (digital) signierten Daten bzw. (digital) signiert ist also zu verstehen, dass die Daten mit einer entsprechenden digitalen Signatur signiert sind.

Unter einer Kopplung ist das Herstellen einer Möglichkeit/ Leitung/ Verbindung zu verstehen, mit der/ über welche Daten sicher übertragen werden können, bevorzugt um eine Therapie von mehreren Infusionspumpen sicher gleichzeitig und/oder nacheinander durchführbar zu machen. Das heißt, dass gekoppelte Infusionspumpen sicher Daten übertragen können/ sicher Daten übertragen, indem sie diese offenbarungsgemäß verschlüsseln und/oder digital signieren, bevorzugt um gemeinsam eine Therapie (eines Patienten) durchzuführen. Die sichere Datenübertragung kann zum Beispiel über Kabel oder kabellos und/oder über zum Beispiel ein internes Krankenhausnetzwerk erfolgen. Ein solches Netzwerk kann auch bereits verschlüsselte Leitungen, also z.B. eine Point-to-Point-Verschlüsselung, bereitstellen oder die Leitungen können unverschlüsselt sein.

Unter den Daten sind zum Beispiel Statusmeldungen, Nachrichten und Instruktionen miteingeschlossen. Eine Instruktion kann zum Beispiel das Starten der Infusionspumpe, das Übernehmen der Therapie oder eine Anweisung, sich mit zumindest einer weiteren und/oder sich mit einer anderen Infusionspumpe zu koppeln bzw. für eine Kopplung anzufragen, sein. Eine Statusmeldung kann z.B. die Menge von einem (noch) vorhandenen Medikament, eine Störungsmeldung oder eine Zustandsmeldung, wie z.B. einsatzbereit, aktiv, im Stand-by oder derzeitige Wartung, sein.

Eine Infusionspumpe, die einer anderen Infusionspumpe (eine) Instruktion(en)/ bzw. Anweisung(en) bzw. Befehl(e) bzw. Aufforderung(en) schickt, kann, insbesondere bei einem Take-Over-Mode, als Master bezeichnet werden. Entsprechend kann die die Instruktion(en) erhaltende Infusionspumpe als Slave bezeichnet werden. Ein Master kann also zum Beispiel eine Therapie durchführen und den Slave anweisen, diese Therapie zu übernehmen.

Es ist zu verstehen, dass die Datenübertragung als sicher bezeichnet wird/ werden kann, da offenbarungsgemäß die Daten mit einer digitalen Signatur versehen/ digital signiert sind/ werden und/oder mit einer Verschlüsselung versehen/ verschlüsselt sind/ werden.

Es ist zu verstehen, dass eine digitale Signatur in der Kryptologie dazu dient, dass der Absender der Daten, die digital signiert sind, damit eindeutig kennzeichnet, dass diese Daten von ihm stammen. In anderen Worten dient die digitale Signatur also dazu, dass ein Empfänger der Daten anhand der digitalen, einmaligen und fälschungssicheren Signatur überprüfen kann, ob die Daten wirklich von dem Absender stammen, oder ob jemand anderes die Daten sendet und/oder die Daten abgegriffen und verändert hat und dann an den Empfänger versendet. Eine digitale Signatur ist also eine Art Ausweis. Die Daten können zum Beispiel basierend auf bekannten digitalen Signaturverfahren, wie z.B. RSA, DAS, El-Gamal oder darauf basierenden Verfahren, oder anderen Verfahren digital signiert/ authentisiert und anhand der digitalen Signatur überprüft / authentifiziert werden. Bevorzugt wird die digitale Signatur basierend auf einem asymmetrischen Kryptosystem erzeugt.

Es ist zu verstehen, dass verschlüsselte Daten in der Kryptografie/ Kryptologie dazu dienen, dass Unbefugte sie nicht entschlüsseln und somit nicht lesen können. Bevorzugt werden die verschlüsselten Daten basierend auf asymmetrischen Verschlüsselungsverfahren bzw. Public-Key-Verschlüsselungsverfahren bzw. asymmetrischen Kryptosystemen, wie z.B. RSA oder darauf basierenden Verfahren, verschlüsselt und entschlüsselt. Die Offenbarung ist aber nicht darauf beschränkt, Daten können auch mittels symmetrischen Verschlüsselungsverfahren verschlüsselt werden.

Es ist zu verstehen, dass offenbarungsgemäß für das Verschlüsseln und Entschlüssen und/oder digital Signieren und Prüfen von digitalen Signaturen ein gemeinsames Verfahren oder mehrere verschiedene Verfahren oder dasselbe Verfahren getrennt/ mehrfach verwendet werden kann.

Es ist weiter zu verstehen, dass Daten, die verschlüsselt sind und/oder digital signiert sind, und die eine der beiden Infusionspumpen erhält, von der anderen der beiden Infusionspumpen stammen kann, oder auch von einer weiteren Infusionspumpe oder einer anderen Vorrichtung, wie zum Beispiel einem Computer. In anderen Worten kann das Infusionspumpensystem auch weitere Infusionspumpen oder andere Vorrichtungen aufweisen und ist nicht auf exakt zwei Infusionspumpen beschränkt.

Unter einem Erhalten von (digital signierten und/oder verschlüsselten Daten) kann auch ein Empfangen, Verarbeiten, Auswerten, Entschlüsseln oder Authentifizieren (der Daten/ Verschlüsselung/ digitalen Signatur) (wie nachfolgend beschrieben) miteingeschlossen sein.

Die Vorteile der Offenbarung bestehen darin, dass zumindest zwei Infusionspumpen untereinander sicher Daten übertragen können bzw. zumindest einseitig sicher Daten erhalten werden. Somit können die zwei Infusionspumpen auf sichere Art und Weiße zusammenarbeiten, um z.B. gemeinsam eine Therapie eines Patienten durchzuführen. Insbesondere für einen Take-Over-Mode ist es wichtig, dass die Daten zuverlässig und sicher übertragen werden, sodass keine Pause während der Therapie entstehen kann. Der Patient ist davor geschützt, dass Daten, die die Infusionspumpen erhalten/ austauschen, manipuliert werden und/oder von einer nicht autorisierten/ fremden Vorrichtung stammen. Des Weiteren ist die offenbarungsgemäße, sichere Datenübertragung für z.B. ein hausinternes und/oder kabelloses Netzwerk, wie WLAN, geeignet, sodass es nicht notwendig ist, dass die Infusionspumpen per Kabel verbunden sind/ werden. Somit werden Komponenten gespart und es ist nicht nötig, die Infusionspumpen per Kabel zu verbinden oder umzustecken. Eine Verbindung/ Kopplung über ein kabelloses Netzwerk ist flexibler. Weiter ist die offenbarungsgemäße sichere Datenübertragung zwischen den Infusionspumpen kostengünstig, da nur die Kommunikation zwischen den Infusionspumpen bzw. zumindest an eine Infusionspumpe/ im Infusionspumpensystem sicher ist und somit nur dort (zumindest einseitig) erhaltene Daten verschlüsselt oder digital signiert werden. Es ist also dadurch nicht nötig, dass alle Infusionspumpen zu jeder Zeit sicher Daten übertragen oder ein ganzes Netzwerk immer verschlüsselt ist.

Bevorzugt sind die Infusionspumpen Spritzenpumpen, sie sind allerdings nicht darauf beschränkt. Es kann sich zum Beispiel auch um volumetrische oder peristaltische Infusionspumpen handeln.

Bevorzugt sendet die erste Infusionspumpe digital signierte Daten bzw. Daten mit digitaler Signatur an die zweite Infusionspumpe und/oder die zweite Infusionspumpe sendet Daten verschlüsselt an die erste Infusionspumpe. Somit kann zumindest einseitig eine sichere Datenübertragung erfolgen. Dies kann bereits ausreichend sein, damit eine Infusionspumpe der anderen eine Anweisung sicher schicken kann z.B. eine Therapie zu übernehmen. Die Datenübertragung(en) kann/ können aber auch wechselseitig/ in beide Richtungen oder in umgekehrte Richtung stattfinden. Das heißt, zusätzlich oder alternativ kann auch die zweite Infusionspumpe digital signierte Daten bzw. Daten mit digitaler Signatur an die erste Infusionspumpe senden und/oder die erste Infusionspumpe kann Daten verschlüsselt an die erste Infusionspumpe senden. In anderen Worten kann das Infusionspumpensystem für eine wechselseitige Übertragung digital signierter Daten und/oder wechselseitige Übertragung verschlüsselter Daten eingerichtet sein und/oder diese durchführen.

Bevorzugt weist das Infusionspumpensystem zumindest zwei Schlüssel zum Entschlüssen und Verschlüsseln von Daten und/oder zum Erzeugen und Prüfen digitaler Signaturen auf. Bei wechselseitiger Übertragung von digital signierten Daten und/oder verschlüsselten Daten kann das Infusionspumpensystem auch vier solcher Schlüssel aufweisen. Das Infusionspumpensystem kann aber auch mehr solcher Schlüssel aufweisen, insbesondere wenn das Infusionspumpensystem mehr als zwei Infusionspumpen oder weitere Vorrichtungen aufweist.

Unter Schlüssel ist in dieser Offenbarung ein digitaler Schlüssel im Sinne der Kryptologie! Kryptografie zu verstehen, und nicht zum Beispiel ein mechanischer.

Bevorzugt hat die erste Infusionspumpe (zumindest) einen ersten Schlüssel und die zweite Infusionspumpe (zumindest) einen zweiten Schlüssel, bevorzugt der zwei Schlüssel wie voranstehend beschrieben, wobei der erste und zweite Schlüssel ein Schlüsselpaar bilden. Unter einem Schlüsselpaar sind bevorzugt zwei Schlüssel zu verstehen, wovon einer eine Datei verschlüsselt und nur der andere/ diese beiden diese Datei dann entschlüsseln kann/ können und/oder einer eine Datei mit einer digitalen Signatur authentisiert und der andere sie authentifiziert.

Dabei können der erste und zweite Schlüssel identisch/ gleich/ eine Kopie voneinander sein, zum Beispiel ein geheimer Schlüssel (secret key) für symmetrische Verschlüsselung, mit dem Daten verschlüsselt und entschlüsselt werden können. Der erste und zweite Schlüssel können aber auch verschieden sein. Zum Beispiel können der erste und zweite Schlüssel ein Schlüsselpaar bilden mit einem privaten und einem öffentlichen Schlüssel (private key, public key) für ein asymmetrisches Kryptoverfahren/- system zum Verschlüsseln-Entschlüsseln und/ oder Authentisieren-Authentifizieren. Beide Infusionspumpen können auch über jeweils zwei Schlüssel für ein wechselseitiges asymmetrisches Kryptoverfahren/- system verfügen.

Bevorzugt erzeugt die erste Infusionspumpe den ersten und zweiten Schlüssel und die zweite Infusionspumpe erhält/ hat den zweiten Schlüssel von der ersten Infusionspumpe. Alternativ oder zusätzlich kann auch die zweite Infusionspumpe einen ersten und einen zweiten Schlüssel erzeugen und den zweiten Schlüssel davon an die erste Infusionspumpe geben.

Bevorzugt ist der erste Schlüssel ein privater Schlüssel, den nur die Infusionspumpe hat, die ihn erstellt, bevorzugt die erste Infusionspumpe, und bevorzugt ist der zweite Schlüssel ein öffentlicher Schlüssel, der an eine Vielzahl von (anderen) Infusionspumpen versendet werden kann, zumindest aber an die/ eine andere Infusionspumpe (des Infusionspumpensystems), bevorzugt die zweite Infusionspumpe, versendet wird. Ein privater Schlüssel wird also nicht versendet und ist nicht für die Öffentlichkeit bestimmt, ein öffentlicher Schlüssel wird versendet/ kann versendet werden.

Bevorzugt bilden der erste, private Schlüssel und der zweite, öffentliche Schlüssel ein Schlüsselpaar im Sinne von asymmetrischen Kryptoverfahren/- sytemen. Dies hat insbesondere den Vorteil, dass die Übertragung des zweiten Schlüssels nicht über eine sichere Leitung/ auf sichere Weise zu erfolgen hat, da er ein öffentlicher Schlüssel ist. Somit kann der zweite Schlüssel übergeben werden, um danach eine sichere Datenübertragung zu ermöglichen, wobei zu diesem Zeitpunkt die Leitung noch nicht sicher zu sein hat/ es nicht notwendig ist, dass es eine sichere Leitung bereits gibt, um Schlüssel auszutauschen.

Bevorzugt ist der erste Schlüssel (private Schlüssel) eingerichtet, Daten, die von seinem Inhaber/ Erzeuger, bevorzugt der ersten Infusionspumpe, versendet werden, mit einer eindeutigen, individuellen, (möglichst) fälschungssicheren, digitalen Signatur zu versehen, um damit zu kennzeichnen, von wem die Daten sind/ stammen. Bevorzugt ist der zweite Schlüssel (öffentlicher Schlüssel) eingerichtet, eine digitale Signatur von Daten zu überprüfen, und die digitale Signatur des ersten Schlüssels zu authentifizieren bzw. anhand der digitalen Signatur zu entscheiden, ob die Daten von dem Inhaber des ersten Schlüssels (privaten Schlüssels) sind/ stammen.

Es ist zu verstehen, dass die Schlüssel selbst nicht diese Funktionen erfüllen zu brauchen, sondern auch der Inhaber des jeweiligen Schlüssels dies mit dem Schlüssel/ mit dessen Hilfe machen kann.

Bevorzugt nimmt der Inhaber des zweiten Schlüssels, bevorzugt die zweite Infusionspumpe, Daten für die der zweite Schlüssel bzw. für die er mit dem zweiten Schlüssel anhand der digitalen Signatur der Daten entscheidet, dass sie von dem Inhaber des ersten Schlüssels, bevorzugt der ersten Infusionspumpe, stammen, an. Bevorzugt verwirft der Inhaber des zweiten Schlüssels, bevorzugt die zweite Infusionspumpe, Daten, für die der zweite Schlüssel bzw. für die er mit dem zweiten Schlüssel anhand der digitalen Signatur der Daten entscheidet, dass sie nicht von dem Inhaber des ersten Schlüssels, bevorzugt der ersten Infusionspumpe, stammen.

Somit verwirft dann die zweite Infusionspumpe z.B. nicht authentifizierte Anweisungen bzw. Anweisungen, die nicht von der authentifizierten, ersten Infusionspumpe stammen. Dadurch kann sichergestellt werden, dass manipulierte Daten und/oder Daten von einer nicht vertrauenswürdigen Quelle die zweite Infusionspumpe nicht täuschen/ manipulieren.

Es ist zu verstehen, dass dies für eine Datenübertragung in dem Infusionspumpensystem bzw. zwischen Infusionspumpen/ an eine Infusionspumpe von einer externen Vorrichtung z.B. Infusionspumpe gilt, aber nicht für eine Eingabe an eine Infusionspumpe. In anderen Worten nehmen die Infusionspumpen natürlich bevorzugt z.B. manuelle, bzw. direkte Eingaben von Anwendern wie Ärzten oder Krankenschwestern an und verwerfen diese nicht. Gleiches gilt bevorzugt auch für (anders/ andere) autorisierte/ authentifizierte Vorrichtungen, zum Beispiel Computer, (andere) Eingabegeräte oder weitere Infusionspumpen.

Unter dem Verwerfen kann ein Ignorieren, Löschen, gar nicht erst Speichern, gar nicht erst Annehmen und gar nicht erst Öffnen der Daten miteingeschlossen sein.

Es ist zu verstehen, dass anhand der digitalen Signatur entschieden werden kann, ob die Daten vom Inhaber des ersten Schlüssels stammen, und damit gemeint ist, dass, wenn die digitale Signatur anders ist oder fehlt, die Daten nicht von dem Inhaber des ersten Schlüssels stammen und/oder die Daten (dazwischen) verändert wurden.

Bevorzugt ist der zweite Schlüssel (alternativ oder zusätzlich) eingerichtet/ in der Lage, Daten so zu verschlüsseln, dass sie (nahezu) ausschließlich/ nur von dem ersten Schlüssel entschlüsselt werden können. Bevorzugt ist (nahezu) ausschließlich/ nur der erste Schlüssel eingerichtet/ in der Lage, Daten, die von dem zweiten Schlüssel verschlüsselt sind/ wurden, zu entschlüsseln. Somit können abgefangene Daten nicht entschlüsselt und manipuliert werden.

Es ist zu verstehen, dass die Schlüssel selbst nicht diese Funktionen erfüllen zu brauchen, sondern auch der Inhaber des jeweiligen Schlüssels dies mit dem Schlüssel/ mit dessen Hilfe machen kann.

Bevorzugt sind die Infusionspumpen (des Infusionspumpensystems/ mögliche bzw. verfügbare Infusionspumpen für das Infusionspumpensystem) eingerichtet, eine Kopplungsinformation zu erzeugen und auszugeben. Ferner sind die Infusionspumpen bevorzugt eingerichtet, die Kopplungsinformation an zumindest eine/ die andere Infusionspumpe zu senden und von einer/ der anderen Infusionspumpe zu empfangen/ erhalten. Die Kopplungsinformation kann zum Beispiel ein Zahlencode, Wort, Farbcode oder Ähnliches sein. Die Offenbarung ist aber nicht darauf beschränkt, es kann sich auch zum Beispiel um ein Audio-Signal handeln. Die Kopplungsinformation wird bevorzugt von einer Infusionspumpe, bevorzugt der ersten Infusionspumpe, erzeugt und an zumindest eine/die andere Infusionspumpe, bevorzugt die zweite Infusionspumpe, verschickt.

Bevorzugt ist zumindest eine Infusionspumpe bzw. sind die Infusionspumpen (des Infusionspumpensystems/ mögliche bzw. verfügbare Infusionspumpen für das Infusionspumpensystem) eingerichtet, (basierend auf der Kopplungsinformation) eine Bestätigung zur Kopplung, vorzugsweise eine manuelle Eingabe eines Anwenders, zu registrieren (und bevorzugt führen sie dann/ nur bei Bestätigung die Kopplung durch bzw. sind dazu eingerichtet). In anderen Worten kann zumindest eine Infusionspumpe bzw. können die Infusionspumpen bevorzugt eine Bestätigung zur Kopplung, die auf der Kopplungsinformation basiert, z.B. durch einen Abgleich der Kopplungsinformation, empfangen und verarbeiten, um dann die Kopplung durchzuführen. Bevorzugt wird also ohne die Bestätigung die Kopplung nicht durchgeführt. In anderen Worten wird bevorzugt die Kopplung, vorzugsweise durch einen Anwender, bestätigt und damit autorisiert. Ein besonders bevorzugtes Ausführungsbeispiel ist dadurch gekennzeichnet, dass die erste Infusionspumpe und die zweite Infusionspumpe eingerichtet sind, eine Kopplungsinformation auszugeben, bevorzugt anzuzeigen, und zumindest eine aus der ersten Infusionspumpe und der zweiten Infusionspumpe eingerichtet ist, bevorzugt beide Infusionspumpen eingerichtet sind, basierend auf der Kopplungsinformation eine Bestätigung zur Kopplung zu registrieren. In anderen Worten kann die Bestätigung zur Kopplung an beiden Infusionspumpen erfolgen oder alternativ nur an einer (Master-)Infusionspumpe.

Die Kopplungsinformation ist eine Information zum Koppeln von zumindest zwei Infusionspumpen miteinander. Ein Anwender, zum Beispiel ein Arzt oder eine Krankenschwester, kann dann die zumindest zwei Infusionspumpen miteinander koppeln, indem er/ sie die Kopplungsinformationen, die die zumindest zwei Infusionspumpen anzeigen, miteinander abgleicht. Wenn die Kopplungsinformationen übereinstimmen, kann der Anwender dies an zumindest einer der Infusionspumpen oder beiden Infusionspumpen bestätigen und damit die Kopplung autorisieren, sodass die Kopplung durchgeführt wird. Somit ist dann eine sichere Datenübertragung möglich und von einem Anwender überprüft und bestätigt. Dies erhöht die Sicherheit. Es ist zu verstehen, dass also bevorzugt, wenn der Anwender die Kopplung autorisiert/ bestätigt, diese durchgeführt wird, und die Schlüssel dazu behalten bzw. angenommen werden und dann zur sicheren Datenübertragung verwendet werden. Wird die Kopplung vom Anwender nicht autorisiert, da z.B. die Kopplungsinformationen nicht übereinstimmen, werden bevorzugt die Schlüssel verworfen/ gelöscht.

Bevorzugt sind die erste und zweite Infusionspumpe (und ggfls. weitere (verfügbare) Infusionspumpen) eingerichtet, miteinander gekoppelt und voneinander entkoppelt zu werden. Bevorzugt werden die Infusionspumpen nach Beendigen einer Therapie eines Patienten entkoppelt, besonders bevorzugt sind sie dazu eingerichtet, dies selbstständig durchzuführen. Bevorzugt werden die jeweiligen Schlüssel beim Entkoppeln verworfen. Bevorzugt kann z.B. die erste Infusionspumpe ihren Schlüssel verwerfen/ löschen und der zweiten Infusionspumpe eine digital signierte Anweisung schicken, dies ebenfalls mit ihrem Schlüssel zu tun.

Bevorzugt sind die erste und zweite Infusionspumpe (und ggfls. weitere (verfügbare) Infusionspumpen) eingerichtet, eine Anfrage/ Anfragen für eine Kopplung zu erstellen, zu versenden, zu empfangen und/oder auszuwerten! zu verarbeiten. Bevorzugt schickt in einem Take-Over-Mode der (anschließende) Slave eine Anfrage an den (anschließenden) Master. Das heißt, bevorzugt fragt die Infusionspumpe für eine Kopplung an, die dann (nach Kopplung) eine Anweisung, vorzugsweise zum Starten bzw. Übernehmen der Therapie, von der anderen Infusionspumpe erhält.

Bevorzugt sind die erste und zweite Infusionspumpe (und ggfls. weitere verfügbare Infusionspumpen) eingerichtet, eine Anfrage für eine Kopplung (automatisch) anzunehmen oder abzulehnen, vorzugsweise je nachdem ob sie verfügbar sind. Unter verfügbaren Infusionspumpen sind zum Beispiel betriebsbereite, solche mit passenden und/oder ausreichenden Medikament versehene und/oder geeignete Infusionspumpen zu verstehen, die gekoppelt werden können/ koppelbar sind/ ein Infusionspumpensystem, vorzugsweise wie voranstehend beschrieben, bilden können. Verfügbar für eine Kopplung kann auch so verstanden werden, dass sie dazu eingerichtet und/oder bereit sind, also zum Beispiel nicht bereits laufen/ aktiv sind. Bevorzugt kann auch ein Anwender auswählen/ entscheiden, welche Infusionspumpen gekoppelt werden und/oder welche eine Anfrage versenden und/oder welche diese annehmen oder ggfs. ablehnen.

Bevorzugt sind die Infusionspumpen eingerichtet, eine Anfrage für eine Kopplung zu stellen, wenn sie in absehbarer Zukunft nicht mehr verfügbar sein werden/ sein werden könnten. Das heißt, dass zum Beispiel eine Infusionspumpe, die zum Beispiel in Zukunft ihr Medikament verabreicht haben wird, deren Medikament zu Neige geht oder die eine Störung/ Problem hat, eine Anfrage für eine Kopplung stellen kann/ stellt.

Besonders bevorzugt sind die Infusionspumpen eingerichtet, eine Anfrage für eine Kopplung zu stellen, wenn sie bereit oder verfügbar sind, und/oder wenn zum Beispiel ein Medikament in die Infusionspumpe eingelegt ist/ wird und/oder, vorzugsweise über ein User Interface, eine Therapie ausgewählt wird.

Bevorzugt ist in einer Anfrage eine Information über eine auszuführende Therapie (bzw. Parameter einer Therapie) enthalten. Bevorzugt enthält die Anfrage zum Beispiel, welches Medikament die Infusionspumpe aufweist/ eingelegt ist und/oder welche Therapie (aus einer Vielzahl von Therapien, für die die Infusionspumpen eingerichtet sind, diese auszuführen) ausgewählt wurde! ist. Es kann zum Beispiel auch in einer Anfrage enthalten sein, welches Medikament und welche Menge davon eine Infusionspumpe bereitstellen/ aufweisen soll.

Die Infusionspumpen können eingerichtet sein, bei einer entsprechenden Eingabe eines Anwenders, eine Anfrage (zur Kopplung) zu versenden. Vorzugsweise kann der Anwender dabei auswählen, an welche Infusionspumpe(n) die Anfrage versendet wird und/oder welche die Anfrage annehmen. Die Infusionspumpen können eingerichtet sein, bei einer entsprechender Eingabe eines Anwenders, eine Anfrage (zur Kopplung) abzulehnen oder anzunehmen.

Bevorzugt sind die Infusionspumpen eingerichtet, ihren Status (bzw. die Statusmeldungen) anzuzeigen, beispielsweise ob sie laufen (Therapie aktiv), pausieren, oder verfügbar sind.

Das Infusionspumpensystem kann ferner eine Steuerungsinstanz aufweisen, die (dazu eingerichtet ist, dass sie) verschlüsselte und/oder digital signierte Daten an zumindest eine aus der ersten und der zweiten Infusionspumpe versendet und/oder von zumindest einer aus der ersten und zweiten Infusionspumpe verschlüsselte und/oder digital signierte Daten empfängt. Die Steuerungsinstanz kann eine Vorrichtung sein, die bevorzugt von einem Anwender des Infusionspumpensystems bedient wird, wie ein Computer, ein Eingabegerät und/oder eine weitere Infusionspumpe. Die Steuerungsinstanz kann auch anstelle der ersten oder zweiten Infusionspumpe des Infusionspumpensystems vorhanden sein und/oder deren Merkmale/ Funktion(en) aufweisen/ übernehmen.

Die Offenbarung betrifft weiterhin ein Verfahren zur sicheren Datenübertragung für ein Infusionspumpensystem, vorzugsweise wie voranstehend beschrieben, mit einer ersten Infusionspumpe und einer zweiten Infusionspumpe, das folgende Schritte, vorzugsweise in dieser Reihenfolge, aufweist:
- Koppeln der ersten und zweiten Infusionspumpe;
- Erhalten von mit einer digitalen Signatur signierten Daten für zumindest eine aus der ersten und zweiten Infusionspumpe; und/oder
- Erhalten von mit einer Verschlüsselung verschlüsselten Daten für zumindest eine aus der ersten und zweiten Infusionspumpe.

Ferner kann der Schritt Koppeln der ersten und zweiten Infusionspumpe Folgendes, vorzugsweise wie voranstehend beschrieben, und/oder folgende Schritte (oder nur Teile davon), vorzugsweise in dieser Reihenfolge, aufweisen:
- Erzeugen eines ersten Schlüssels und eines zweiten Schlüssels von der ersten Infusionspumpe;
- Erzeugen und Ausgeben einer Kopplungsinformation, vorzugsweise an einen Anwender des Infusionspumpensystems, von der ersten Infusionspumpe;
- Übertragen der Kopplungsinformation und des zweiten Schlüssels an die zweite Infusionspumpe;
- Ausgeben der Kopplungsinformation von der zweiten Infusionspumpe, vorzugsweise an den Anwender des Infusionspumpensystems;
- Überprüfen der Kopplungsinformation, angezeigt von der ersten Infusionspumpe und der zweiten Infusionspumpe, vorzugsweise durch den Anwender des Infusionspumpensystems;
- Bei identischer Kopplungsinformation, Bestätigung dieser, vorzugsweise durch den Anwender (19) des Infusionspumpensystems (20), und Kopplung der ersten Infusionspumpe und der zweiten Infusionspumpe für ein Erhalten von Daten mit einer digitalen Signatur für zumindest eine aus der ersten und zweiten Infusionspumpe und/oder ein Erhalten von verschlüsselten Daten für zumindest eine aus der ersten und zweiten Infusionspumpe.

Es ist zu verstehen, dass also bevorzugt, wenn (der Anwender die Kopplung autorisiert), die Kopplung durchgeführt wird, und die Schlüssel dazu behalten bzw. angenommen werden und dann zur sicheren Datenübertragung verwendet werden. Wird die Kopplung vom Anwender nicht autorisiert/ nicht (vollständig) durchgeführt, da z.B. die Kopplungsinformationen nicht übereinstimmen, werden die Schlüssel bevorzugt verworfen/ gelöscht.

Es ist zu verstehen, dass das Koppeln der ersten und zweiten Infusionspumpe auch ein Koppeln von mehr als zwei Infusionspumpen und/oder mit zumindest einer Steuerungsinstanz, bevorzugt wie voranstehend beschrieben, beinhalten kann. Es können mehrere Kopien des zweiten Schlüssels erstellt werden, und diese an mehrere Infusionspumpen und/oder die zumindest eine Steuerungsinstanz versendet werden, sodass es z.B. mehrere Infusionspumpen geben kann, die die Funktionen und Merkmale der zweiten Infusionspumpe aufweisen. Es kann auch die Kopplungsinformation mehrfach erzeugt und verschickt werden oder die Kopplung z.B. mehrmals mit verschiedenen Partnern nacheinander durchgeführt werden. Eine Infusionspumpe kann auch mit mehreren Infusionspumpen gleichzeitig gekoppelt sein.

Es ist ferner zu verstehen, dass auch wechselseitig zwei Schlüsselpaare mit jeweils einem ersten und zweiten Schlüssel ausgetauscht werden können, dass es also einen Schritt/ Schritte gibt mit:
- Erzeugen eines (weiteren) ersten Schlüssels und eines zweiten Schlüssels (eines weiteren Schlüsselpaares) von der zweiten
   Infusionspumpe;
- Übertragen dieses zweiten Schlüssels an die erste Infusionspumpe.

In anderen Worten können beide/ alle Infusionspumpen ein Schlüsselpaar aus einem ersten und zweiten Schlüssel erstellen, und den zweiten Schlüssel an (eine) andere Infusionspumpe(n) versenden.

Es ist zu verstehen, dass der voranstehend beschriebene Schritt des Koppelns, bevorzugt wie voranstehend beschrieben, selbst Schritte aufweist/ aufweisen kann, die als ein Verfahren zum Koppeln von (zumindest) einer ersten und einer zweiten Infusionspumpe, bevorzugt wie voranstehend beschrieben, angesehen werden können. In anderen Worten kann der Schritt Koppeln der ersten und zweiten Infusionspumpe auch ein Verfahren zum Koppeln (zumindest) einer ersten und zweiten Infusionspumpe sein. Dieser Schritt/ Dieses Verfahren des Koppelns kann bevorzugt durchgeführt und abgeschlossen werden, bevor ein Patient an eine der (gekoppelt werdenden/ gekoppelten) Infusionspumpen angeschlossen wird, und ist somit dann kein Verfahren zur chirurgischen oder therapeutischen Behandlung des menschlichen oder tierischen Körpers und kein Diagnostizierverfahren, das am menschlichen oder tierischen Körper vorgenommen wird.

Ferner ist zu verstehen, dass der Schritt/ das Verfahren des Koppelns sowohl einmal initial vor Datenübertragung(en) (bis zu einem Entkoppeln) stattfinden kann als auch öfters oder vor jeder Datenübertragung.

Das Verfahren zur sicheren Datenübertragung für ein Infusionspumpensystem und/oder der Schritt/ das Verfahren des Koppelns kann folgenden Schritt (vorgeordnet) aufweisen:
- Anfragen der zweiten Infusionspumpe bei der ersten Infusionspumpe für eine/ die Kopplung/ zum Koppeln und/oder Anfragen der zweiten Infusionspumpe bei einer Vielzahl von verfügbaren Infusionspumpen nach einer ersten Infusionspumpe für Kopplung und Auswahl der ersten Infusionspumpe aus der Vielzahl von verfügbaren Infusionspumpen.

Ferner kann das Verfahren zur sicheren Datenübertragung für ein Infusionspumpensystem und/oder der Schritt/ das Verfahren des Koppelns folgenden Schritt aufweisen:
- Annehmen (oder Ablehnen) der Anfrage der zweiten Infusionspumpe bei der ersten Infusionspumpe für eine/ die Kopplung/ zum Koppeln von der ersten Infusionspumpe.

Bei Annahme können dann die anderen/ weitere/ die Schritte des Verfahrens zur sicheren Datenübertragung für ein Infusionspumpensystem und/oder des Schrittes/ des Verfahrens des Koppelns, wie voranstehend beschrieben, erfolgen.

Es ist zu verstehen, dass also bevorzugt eine Anfrage für eine Kopplung zunächst zwischen (zumindest) zwei Infusionspumpen erfolgt, und wenn dann, in diesem Fall von der ersten Infusionspumpe, die Anfrage angenommen wird, anschließend die Kopplung, in diesem Fall zwischen erster und zweiter Infusionspumpe, durchgeführt wird und damit (erst dann) eine sichere Datenübertragung ermöglicht wird. Daher wird bevorzugt die Kopplung von einem Anwender, vorzugsweise mit der Kopplungsinformation, bestätigt und autorisiert, um sicherzugehen, dass eine Anfrage, die aufgrund der zu diesem Zeitpunkt noch nicht sicheren Datenübertragung z.B. abgefangen und verfälscht werden könnte, nicht automatisch zu einer Kopplung führt.

Es kann eine Vielzahl von Infusionspumpen angefragt werden, um gekoppelt zu werden! ein Infusionspumpensystem, vorzugsweise wie voranstehend beschrieben, zu bilden. Unter verfügbaren Infusionspumpen sind zum Beispiel betriebsbereite, solche mit passendem Medikament versehene und/oder geeignete Infusionspumpen zu verstehen, die gekoppelt werden können/ koppelbar sind/ ein Infusionspumpensystem, vorzugsweise wie voranstehend beschrieben, bilden können. Verfügbar für eine Kopplung kann hier auch so verstanden werden, dass sie dazu eingerichtet und/oder bereit sind. Es kann auch die erste Infusionspumpe bei der zweiten Infusionspumpe bzw. einer Vielzahl von Infusionspumpen anfragen.

Das Verfahren zur sicheren Datenübertragung für ein Infusionspumpensystem kann weiter Folgendes/ folgende (nachgelagerte) Schritte, vorzugsweise in dieser Reihenfolge, aufweisen:
- Versenden von Daten mit einer eindeutigen und individuellen digitalen Signatur, die von dem ersten Schlüssel erstellt wird, von der ersten Infusionspumpe;
- Überprüfen der digitalen Signatur von Daten mit dem zweiten Schlüssel von der zweiten Infusionspumpe und Entscheidung anhand der digitalen Signatur, ob die Daten von der ersten Infusionspumpe stammen;
- Annehmen der Daten durch die zweite Infusionspumpe, wenn die Entscheidung ergibt, dass die Daten von der ersten Infusionspumpe stammen; oder
- Verwerfen der Daten durch die zweite Infusionspumpe, wenn die Entscheidung ergibt, dass die Daten nicht von der ersten Infusionspumpe stammen;
   und/oder Folgendem/ folgenden weiteren Schritten:
- Verschlüsseln von Daten mit dem zweiten Schlüssel von der zweiten Infusionspumpe;
- Versenden der verschlüsselten Daten von der zweiten Infusionspumpe;
- Erhalten der verschlüsselten Daten von der ersten Infusionspumpe;
- Entschlüsseln der Daten mit dem ersten Schlüssel von der ersten Infusionspumpe

Wie voranstehend beschrieben, können auch wechselseitig oder in andere Richtung verschlüsselte Daten und/ oder wechselseitig digital signierte Daten übertragen werden.

Dementsprechend können die erste und zweite Infusionspumpe (in Verfahrensschritten) auch ausgetauscht werden und/oder die zweite Infusionspumpe zusätzlich Merkmale/ Funktionen/ Schritte der ersten Infusionspumpe aufweisen/ ausführen und die erste Infusionspumpe zusätzlich Merkmale/ Funktionen/Schritte der ersten Infusionspumpe aufweisen/ ausführen.

Das Verfahren zur sicheren Datenübertragung für ein Infusionspumpensystem und/oder der Schritt/ das Verfahren des Koppelns kann folgenden Schritt (zum Ende) aufweisen:
- Entkoppeln von den gekoppelten Infusionspumpen (durch einen/ den Anwender oder selbstständig, bevorzugt wie voranstehend beschrieben).

Der Schritt kann ebenfalls stattfinden, wenn kein Patient an die Infusionspumpen (mehr) angeschlossen ist. Bevorzugt findet das Entkoppeln statt, wenn eine Therapie eines Patienten beendet ist. Bevorzugt werden die jeweiligen Schlüssel beim Entkoppeln verworfen.

Die Offenbarung betrifft weiterhin ein computerlesbares Speichermedium, das Funktionen aufweist, die ein Infusionspumpensystem, vorzugsweise wie voranstehend beschrieben, veranlassen, (die) Schritte eines Verfahrens zur sicheren Datenübertragung für ein Infusionspumpensystem und/oder eines Verfahrens zum Koppeln bzw. des Koppelns, vorzugsweise wie voranstehend beschrieben, auszuführen.

### Kurzbeschreibung der Figuren

Die Offenbarung wird im Folgenden anhand bevorzugter Ausführungsformen und unter Bezugnahme auf die beigefügten Figuren näher erläutert.
Fig. 1 zeigt ein Anfragen einer Infusionspumpe für eine Kopplung für eine sichere Datenübertragung gemäß der vorliegenden Offenbarung;
Fig. 2 zeigt ein Koppeln zweier Infusionspumpen für eine sichere Datenübertragung gemäß der vorliegenden Offenbarung;
Fig. 3 zeigt ein Übertragen digital signierter Daten zwischen zwei Infusionspumpen gemäß der vorliegenden Offenbarung;
Fig. 4 zeigt ein Übertragen verschlüsselter Daten zwischen zwei Infusionspumpen gemäß der vorliegenden Offenbarung.
Fig. 5 zeigt ein wechselseitiges Übertragen digital signierter Daten zwischen zwei Infusionspumpen gemäß der vorliegenden Offenbarung.
Fig. 6 zeigt ein wechselseitiges Übertragen verschlüsselter Daten zwischen zwei Infusionspumpen gemäß der vorliegenden Offenbarung.
Fig. 7 zeigt ein Infusionspumpensystem mit einer Steuerungsinstanz und sicherer Datenübertragung gemäß der vorliegenden Offenbarung;
Fig. 8 zeigt weitere, sichere Datenübertragungen zu Fig. 7;
Fig. 9 zeigt ein Anfragen einer Infusionspumpe für eine Kopplung für eine sichere Datenübertragung gemäß der vorliegenden Offenbarung;
Fig. 10 entspricht Fig. 2;
Fig. 11 entspricht Fig. 3;
Fig. 12 zeigt ein Anfragen einer Infusionspumpe aus Fig. 9 bei einer anderen Infusionspumpe für eine Kopplung für eine sichere Datenübertragung gemäß der vorliegenden Offenbarung;
Fig. 13 zeigt ein computerlesbares Speichermedium.

Die Figuren sind schematisch und dienen lediglich dem Verständnis der Offenbarung. Die Merkmale der verschiedenen Ausführungsformen können untereinander ausgetauscht werden.

### Detaillierte Beschreibung der bevorzugten Ausführungsformen

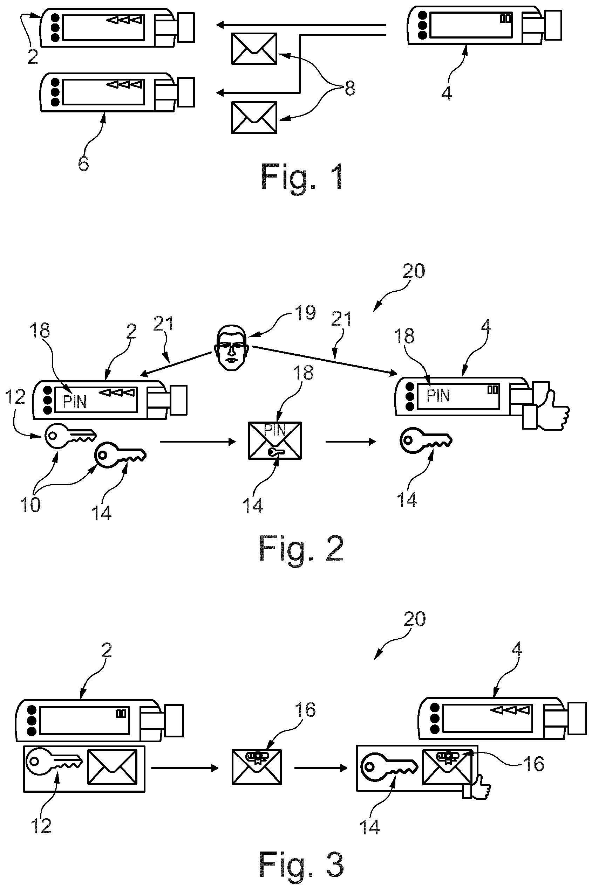

In Fig. 1 wird eine erste Infusionspumpe 2 von einer zweiten Infusionspumpe 4 angefragt für eine Kopplung für eine sichere Datenübertragung. Die zweite Infusionspumpe 4 fragt auch eine dritte Infusionspumpe 6 an. Die zweite Infusionspumpe 4 fragt bei den verfügbaren Infusionspumpen an, in diesem Fall den zwei anderen. Dazu sendet sie eine Anfrage 8 an die verfügbaren Infusionspumpen. Hier nimmt die erste Infusionspumpe 2 die Anfrage 8 der zweiten Infusionspumpe 4 an.

Fig. 2 zeigt die erste Infusionspumpe 2 und zweite Infusionspumpe 4 aus Fig. 1. Die erste Infusionspumpe 2 erzeugt ein (digitales, kryptologisches/ kryptografisches) Schlüsselpaar 10 mit einem ersten Schlüssel 12 und einem zweiten Schlüssel 14. Der erste Schlüssel 12 ist hier ein privater Schlüssel (private key) und eingerichtet, Daten, die von der ersten Infusionspumpe 2 versendet werden, mit einer eindeutigen und individuellen, fälschungssicheren digitalen Signatur 16 zu versehen, um zu kennzeichnen, dass sie von der ersten Infusionspumpe 2 stammen. Der zweite Schlüssel 14 ist hier ein öffentlicher Schlüssel (public key), der eingerichtet ist, eine digitale Signatur 16 von Daten, (die an die zweite Infusionspumpe 4 gesendet werden), zu prüfen und zu entscheiden, ob die digitale Signatur 16 von dem ersten Schlüssel 12 ist und die Daten somit von der ersten Infusionspumpe 2 stammen oder ob sie verändert wurden oder von wo anders aus verschickt wurden. Das Schlüsselpaar 10 ist hier also asymmetrisch bzw. für ein asymmetrisches Kryptoverfahren/ -sytem. Die erste Infusionspumpe 2 erzeugt eine Kopplungsinformation 18 "PIN" und gibt diese für einen Anwender 19 z.B. einen Arzt oder eine Krankenschwester aus/ zeigt sie an. Dann oder dabei schickt die erste Infusionspumpe 2 die Kopplungsinformation 18 und den zweiten Schlüssel 14 an die zweite Infusionspumpe 4. Die zweite Infusionspumpe 4 hat jetzt den zweiten Schlüssel 14 und die Kopplungsinformation 18, und gibt die Kopplungsinformation 18 für den Anwender 19 aus/ zeigt sie an. Der Anwender 19 gleicht die von den Infusionspumpen 2, 4 angezeigten Kopplungsinformationen 18 miteinander ab. Da sie identisch sind/ übereinstimmen, führt er eine Bestätigung 21 durch und autorisiert damit die Kopplung, woraufhin die erste Infusionspumpe 2 und zweite Infusionspumpe 4 miteinander gekoppelt sind, das heißt, dass sie digital signierte Daten und/oder verschlüsselte Daten mit den vorliegenden Schlüsseln sicher übertragen können/ übertragen, um eine Therapie eines Patienten gemeinsam gleichzeitig oder nacheinander durchzuführen. Die erste und zweite Infusionspumpe 2, 4 sind also Teil eines/ bilden ein Infusionspumpensystem 20.

Fig. 3 zeigt das Infusionspumpensystem 20 aus Fig. 2 und veranschaulicht das Übertragen von digital signierten Daten zur sicheren Datenübertragung. Die erste Infusionspumpe 2 signiert die Daten mit dem ersten Schlüssel 12 und sendet diese dann an die zweite Infusionspumpe 4. Diese prüft die digitale Signatur 16 der Daten mit dem zweiten Schlüssel 14 darauf, ob diese digitale Signatur 16 von dem ersten Schlüssel 12 ist und damit die Daten eindeutig und unverfälscht von der ersten Infusionspumpe 2 stammen. Hier ist dies der Fall und die Daten werden von der zweiten Infusionspumpe 4 angenommen. Ansonsten würden sie verworfen werden.

Die Fig. 1, 2 und 3 zeigen gemeinsam einen Take-Over-Mode mit sicherer Datenübertragung gemäß der vorliegenden Ausführungsform. Zunächst führt die erste Infusionspumpe 2 in Fig. 1 eine bestimmte Therapie eines Patienten mit einem bestimmten Medikament durch (veranschaulicht durch die drei Pfeile, d.h. sie ist aktiv/ läuft). Die zweite Infusionspumpe 4 wird/ ist vorbereitet, eine Therapie durchzuführen, in diesem Fall die Therapie der ersten Infusionspumpe 2 mit demselben Medikament, bzw. diese zu übernehmen. (Es wird z.B. von einem Arzt an der zweiten Infusionspumpe 4 die von dieser durchzuführende Therapie, das Medikament usw. ausgewählt). Die zweite Infusionspumpe 4 ist zunächst nicht aktiv (visualisiert durch die zwei Striche bzw. das Pause-Symbol in Fig. 1). Die zweite Infusionspumpe 4 verschickt die Anfrage 8, in der sie Parameter mitteilt, also für welche Therapie sie vorbereitet ist (welches Medikament sie aufweist/ ausgewählt wurde, welche Therapie ausgewählt wurde usw.). Die erste Infusionspumpe 2 führt in diesem Fall die entsprechende/ diese/ dieselbe Therapie aus, die dritte Infusionspumpe 6 eine andere (beide sind aktiv, dargestellt durch die drei Pfeile, d.h. die Infusionspumpen laufen). Das heißt, die erste Infusionspumpe 2 erkennt anhand der Anfrage 8 (und der darin enthaltenen Parameter), dass die zweite Infusionspumpe 4 vorbereitet geeignet ist, die Durchführung der Therapie zu übernehmen. Darauf erfolgt, wie voranstehend beschrieben und in Fig. 2 gezeigt, die Kopplung der ersten Infusionspumpe 2 und der zweiten Infusionspumpe 4. Dann sendet die erste Infusionspumpe 2, wie in Fig. 3 gezeigt und dazu beschrieben, digital signierte Daten an die zweite Infusionspumpe 4. In diesem Fall handelt es sich um eine Anweisung/ einen Befehl zu starten, also die Durchführung der Therapie zu übernehmen. Die zweite Infusionspumpe 4 übernimmt jetzt die Therapie und verabreicht ihr Medikament, d.h. sie ist jetzt aktiv (dargestellt durch die drei Pfeile) und die erste Infusionspumpe 2 pausiert/ ist nicht mehr aktiv. Es ist somit sichergestellt, dass die Therapie aufrechterhalten wird/ werden kann, es also keine Unterbrechung gibt bzw. die Übergänge zwischen den Infusionspumpen nahtlos erfolgen und die Daten sicher und zuverlässig zwischen den Infusionspumpen ausgetauscht werden können. Die zweite Infusionspumpe 4, die hier die Anfrage 8 stellt, ist ein Slave, dem von dem Master, hier der ersten Infusionspumpe 2, digital signierte Anweisung(en), in diesem Fall der Befehl zum Starten, geschickt werden. Der Master schickt zum Beispiel den Befehl zum Starten, wenn er sein Medikament (nahezu vollständig/ in absehbarer Zeit) verabreicht hat. Der Slave übernimmt dann die Therapie vom Master.

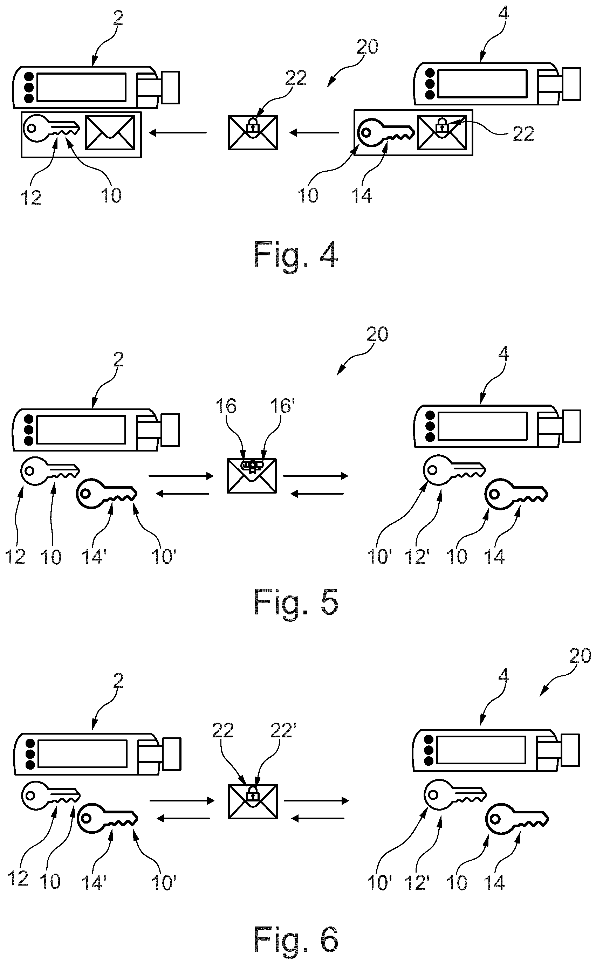

Fig. 4 zeigt das Übertragen von verschlüsselten Daten. Fig. 4 zeigt das Infusionspumpensystem 20 aus Fig. 3, allerdings ist der zweite Schlüssel 14 zusätzlich oder alternativ eingerichtet, Daten zu verschlüsseln, sodass sie nur mit dem ersten Schlüssel 12 entschlüsselt werden können. Der erste Schlüssel 12 ist entsprechend (einzig) eingerichtet, Daten, die mit dem zweiten Schlüssel 14 verschlüsselt wurden, zu entschlüsseln. Das Schlüsselpaar 10 ist hier also ebenfalls ein asymmetrisch bzw. für ein asymmetrisches Kryptoverfahren/ -system, wobei der erste Schlüssel 12 ebenfalls der private Schlüssel (private key) und der zweite Schlüssel 14 ebenfalls der öffentliche Schlüssel (public key) ist. Die zweite Infusionspumpe 4 verschlüsselt Daten, die sie an die erste Infusionspumpe 2 sendet mit einer Verschlüsselung 22, sodass die Daten nur von dem ersten Schlüssel 12 bzw. der ersten Infusionspumpe 2 mit dem ersten Schlüssel 12 entschlüsselt werden können. Die erste Infusionspumpe 2 entschlüsselt dann die Verschlüsselung 22 und kann die Daten lesen und verarbeiten.

Fig. 5 zeigt das Infusionspumpensystem 20 aus Fig. 3, allerdings erzeugt die zweite Infusionspumpe 4 ebenfalls ein zweites Schlüsselpaar 10` mit einem ersten digitalen Schlüssel 12' und einem zweiten digitalen Schlüssel 14`. Den zweiten Schlüssel 14' versendet die zweite Infusionspumpe 4 bei der Kopplung zunächst an die erste Infusionspumpe 2, sodass jetzt die erste Infusionspumpe 2 den zweiten Schlüssel 14' des zweiten Schlüsselpaares 10' hat. Der erste Schlüssel 12' ist hier ein privater Schlüssel (private key) und eingerichtet, Daten, die von der zweiten Infusionspumpe 4 versendet werden, mit einer eindeutigen und individuellen, (fälschungssicheren) digitalen Signatur 16' zu versehen, um zu kennzeichnen, dass sie von der zweiten Infusionspumpe 4 stammen. Der zweite Schlüssel 14' ist hier ein öffentlicher Schlüssel (public key), der eingerichtet ist, eine digitale Signatur 16` von Daten, (die an die erste Infusionspumpe 2 gesendet werden), zu prüfen und zu entscheiden, ob die digitale Signatur 16' von dem ersten Schlüssel 12' ist und die Daten somit von der zweiten Infusionspumpe 4 stammen oder ob sie verändert wurden oder von wo anders aus verschickt wurden. Das Schlüsselpaar 10` ist hier also asymmetrisch bzw. für ein asymmetrisches Kryptoverfahren/ -system. Somit können beide Infusionspumpen 2 bzw. 4 wechselseitig Daten mit (eindeutiger und fälschungssicher) digitaler Signatur 16 bzw. 16' mit dem jeweiligen ersten Schlüssel 12 bzw. 12' versehen und versenden, und die jeweils andere Infusionspumpe 4 bzw. 2 die jeweilige Signatur mit dem jeweiligen zweiten Schlüssel 14 bzw. 14' prüfen und damit sicherstellen, dass die Daten wirklich von der anderen Infusionspumpe 4 bzw. 2 stammen. Dies wird, wie in Fig. 5 veranschaulicht, durchgeführt.

Fig. 6 zeigt das Infusionspumpensystem 20 aus Fig. 5. Allerdings sind zusätzlich oder alternativ die zweiten Schlüssel 14, 14' eingerichtet, Daten jeweils so mit einer (individuellen) Verschlüsselung 22 bzw. 22' zu versehen, dass diese nur von dem entsprechenden ersten Schlüssel 12 bzw. 12' des jeweiligen Schlüsselpaares 10 bzw. 10' entschlüsselt werden kann. (Einzig) Die jeweiligen ersten Schlüssel 12,12` sind entsprechend eingerichtet, Daten, die von dem jeweiligen/ zugehörigen zweiten Schlüssel 14, 14' verschlüsselt wurden, zu entschlüsseln. Somit können die erste Infusionspumpe 2 und die zweite Infusionspumpe 4 sicher verschlüsselte Daten wechselseitig übertragen und tun dies auch, wobei die Schlüssel entsprechend (verwendet werden, um zu) verschlüsseln und entschlüsseln. Die Schlüsselpaare 10 und 10' sind also asymmetrisch bzw. für ein asymmetrisches Kryptoverfahren/ -system.

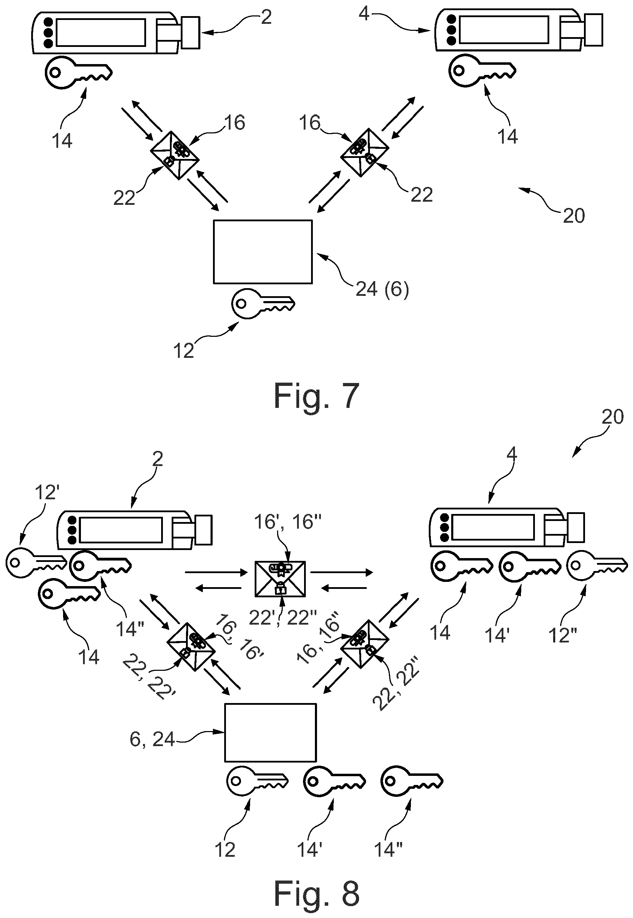

Fig. 7 zeigt ein Infusionspumpensystem 20 mit zwei Infusionspumpen 2, 4 und einer Steuerungsinstanz 24. Die Steuerungsinstanz 24 kann dabei die Funktion und/oder Merkmale der ersten und/oder zweiten Infusionspumpen 2, 4 aus einer der/den vorherigen Figuren aufweisen. Die Steuerungsinstanz 24 kann auch selbst eine dritte Infusionspumpe 6 sein. In diesem Fall ist die Steuerungsinstanz 24 mit den zwei Infusionspumpen 2, 4 gekoppelt. Sie hat selbst einen ersten, privaten Schlüssel 12, der die Merkmale aus den/ einer der vorherigen Figuren aufweist/ aufweisen kann, und die Infusionspumpen 2, 4 haben beide jeweils einen zugehörigen zweiten Schlüssel 14, der die Merkmale aus den vorherigen Figuren aufweist/ aufweisen kann. Somit kann die Steuerungsinstanz 24 digital signierte/ mit einer eindeutigen, fälschungssicheren digitalen Signatur 16 Daten an eine und/oder beide Infusionspumpen 2, 4 schicken, um diesen zum Beispiel sicher Anweisungen zu senden und diese können die digitale Signatur 16 prüfen, wie zuvor beschrieben. Dies wird auch so durchgeführt. Zusätzlich oder alternativ können die oder eine Infusionspumpe(n) 2, 4 sicher verschlüsselte Daten/ Daten mit einer Verschlüsselung 22 an die Steuerungsinstanz 24 senden und nur diese sie mit dem ersten Schlüssel 12 entschlüsseln, wie zuvor beschrieben. Dies wird ebenfalls so durchgeführt.

Fig. 8 zeigt eine wechselseitige sichere Datenübertragung in einem Infusionspumpensystem 20 mit zwei Infusionspumpen 2, 4 und einer Steuerungsinstanz 24, wobei die Steuerungsinstanz 24 auch eine dritte Infusionspumpe 6 sein kann. Es können (und werden) zwischen allen Vorrichtungen des Infusionspumpensystems 20 (Infusionspumpen 2 und 4, 6 oder Steuerungsinstanz 24) sicher jeweils wechselseitig mit einer Verschlüsselung 22 bzw. 22' bzw. 22" verschlüsselte Daten und/oder mit einer digitalen Signatur 16 bzw. 16' bzw. 16" signierte Daten verschickt (werden). Jede der Vorrichtungen des Infusionspumpensystems 20 hat dazu einen privaten ersten Schlüssel 12 bzw. 12' bzw. 12" und jeweils zwei öffentliche, zweite Schlüssel 14` und 14" bzw. 14 und 14" bzw. 14 und 14' zugehörig zu den ersten Schlüsseln der anderen (beiden) Vorrichtungen.

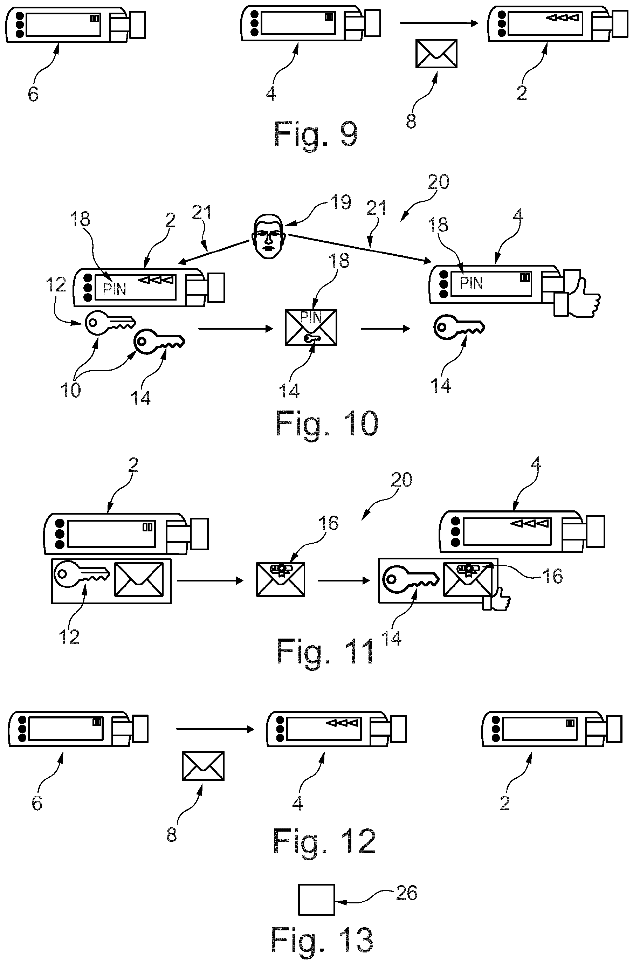

Fig. 9 entspricht Fig. 1, wobei die dritte Infusionspumpe 6 hier beispielhaft nicht eine (andere) Therapie ausführt, sondern nicht aktiv ist. Die zweite Infusionspumpe 4 stellt die Anfrage 8 auch an die dritte Infusionspumpe 6 (nicht dargestellt), sie lehnt diese aber ab.

Fig. 10 entspricht Fig. 2 und zeigt die Kopplung der ersten Infusionspumpe 2 mit der zweiten Infusionspumpe 4.

Fig. 11 entspricht Fig. 3 und zeigt, dass die erste Infusionspumpe 2 der zweiten Infusionspumpe 4 digital signiert die Anweisung schickt, zu starten und die Therapie zu übernehmen.

Fig. 12 zeigt das Anfragen der dritten Infusionspumpe 6 für eine Kopplung bei der zweiten Infusionspumpe 4. Die dritte Infusionspumpe 6 ist jetzt verfügbar und eingerichtet/ bereit, die Therapie von der zweiten Infusionspumpe 4 zu übernehmen, beispielsweise hat der Anwender 19 gerade zuvor (während die zweite Infusionspumpe 4 die Therapie durchführte), (das passende) Medikament eingefüllt oder ausgewählt oder eine (bestimmte) Therapie ausgewählt und somit stellt die dritte Infusionspumpe 6 jetzt die Anfrage 8.

Die Fig. 9 bis 12 veranschaulichen also einen Take-Over-Mode, wobei insgesamt drei Infusionspumpen 2, 4 und 6 eine Therapie gemeinsam nacheinander ausführen. Zunächst macht dies die erste Infusionspumpe 2 in Fig. 9. Die zweite Infusionspumpe 4 fragt für eine Kopplung die erste Infusionspumpe 2 an. Die erste Infusionspumpe 2 und zweite Infusionspumpe 4 werden in Fig. 10 gekoppelt. Die erste Infusionspumpe 2 als Master gibt dann digital signiert und somit sicher den Befehl zu Starten an die zweite Infusionspumpe 4, den Slave. Dann führt die zweite Infusionspumpe 4 die Therapie durch. In Fig. 12 ist die dritte Infusionspumpe 6 bereit, die Therapie fortzuführen, und fragt die zweite Infusionspumpe 4 für eine Kopplung an. Anschließend werden die zweite Infusionspumpe 4 und die dritte Infusionspumpe 6 gekoppelt und die zweite Infusionspumpe 4 weist als Master die dritte Infusionspumpe 6 als Slave an, zu Starten und somit die Therapie zu übernehmen (nicht mehr dargestellt). Somit wird die Therapie zunächst von der ersten Infusionspumpe 2, dann der zweiten Infusionspumpe 4 und dann der dritten Infusionspumpe 6 ausgeführt. Es wird somit auch der Master-Status von der ersten Infusionspumpe 2 an die zweite Infusionspumpe 4 und dann an die dritte Infusionspumpe 6 übergeben und entsprechend wird der Salve-Status von der zweiten Infusionspumpe 4 auf die dritte Infusionspumpe 6 übergeben. Dabei wird immer der (vorherige) Slave zum (neuen) Master. Es können somit auch beliebig viele Infusionspumpen (auch mehr als drei) aneinander gereiht/ geschalten, eine Therapie gemeinsam durchführen und dabei die Durchführung einer Therapie an die nächste Infusionspumpe weiter übergeben.

Fig. 13 zeigt ein computerlesbares Speichermedium 26, das Funktionen aufweist, die ein Infusionspumpensystem 20, vorzugsweise wie voranstehend beschrieben, veranlassen, (die) Schritte eines Verfahrens zur sicheren Datenübertragung für ein Infusionspumpensystem 20, vorzugsweise wie voranstehend beschrieben, und/oder eines Verfahrens zum Koppeln bzw. des Koppelns, vorzugsweise wie voranstehend beschrieben, auszuführen.

### Liste der Bezugszeichen

- 2: erste Infusionspumpe
- 4: zweite Infusionspumpe
- 6: dritte Infusionspumpe
- 8: Anfrage
- 10, 10`, 10": Schlüsselpaar
- 12, 12`, 12": erster Schlüssel
- 14, 14`, 14": zweiter Schlüssel
- 16, 16', 16": digitale Signatur
- 18: Kopplungsinformation
- 19: Anwender
- 20: Infusionspumpensystem
- 21: Bestätigung
- 22, 22`, 22": Verschlüsselung
- 24: Steuerungsinstanz
- 26: computerlesbares Speichermedium

## Patentansprüche

1. Infusionspumpensystem (20), das eine erste Infusionspumpe (2) und eine zweite Infusionspumpe (4) aufweist, die miteinander für eine sichere Datenübertragung gekoppelt sind oder koppelbar sind, wobei das Infusionspumpensystem (20) eingerichtet ist, dass
zumindest eine aus der ersten und der zweiten Infusionspumpe (2, 4) mit einer digitalen Signatur (16) signierte Daten erhält und/oder
zumindest eine aus der ersten und der zweiten Infusionspumpe (2, 4) mit einer Verschlüsselung (22) verschlüsselte Daten erhält.

2. Infusionspumpensystem (20) nach Anspruch 1, wobei die erste Infusionspumpe (2) und/oder die zweite Infusionspumpe (4) eingerichtet ist bzw. sind, eine Kopplungsinformation (18) auszugeben, bevorzugt anzuzeigen, und wobei die erste Infusionspumpe (2) und/oder die zweite Infusionspumpe (4) ferner eingerichtet ist bzw. sind, basierend auf der Kopplungsinformation (18) eine Bestätigung (21) zur Kopplung zu registrieren.

3. Infusionspumpensystem (20) nach Anspruch 1 oder 2, welches eingerichtet ist, dass
die erste Infusionspumpe (2) Daten mit einer digitalen Signatur (16) an die zweite Infusionspumpe (4) versendet und/oder
die zweite Infusionspumpe (4) Daten verschlüsselt an die erste Infusionspumpe (2) versendet.

4. Infusionspumpensystem (20) nach einem der Ansprüche 1 bis 3, welches zumindest zwei Schlüssel (12, 14) zum Entschlüssen und Verschlüsseln von Daten und/oder zum Erzeugen und Prüfen digitaler Signaturen (16) aufweist.

5. Infusionspumpensystem (20) nach Anspruch 4, welches eingerichtet ist, dass zur Kopplung für die sichere Datenübertragung die erste Infusionspumpe (2) einen ersten Schlüssel (12) und die zweite Infusionspumpe (4) einen zweiten Schlüssel (14) der zwei Schlüssel hat, wobei vorzugsweise die erste Infusionspumpe (2) den ersten Schlüssel (12) und den zweiten Schlüssel (14) erzeugt und die zweite Infusionspumpe (4) den zweiten Schlüssel (14) von der ersten Infusionspumpe (2) hat.

6. Infusionspumpensystem (20) nach Anspruch 5, welches eingerichtet ist, dass
der erste Schlüssel (12) ein privater Schlüssel ist, den nur die erste Infusionspumpe (2) hat, und
der zweite Schlüssel (14) ein öffentlicher Schlüssel ist, der an eine Vielzahl von Infusionspumpen versendet werden kann, zumindest aber an die zweite Infusionspumpe (4) von der ersten Infusionspumpe (2) versendet wird.

7. Infusionspumpensystem (20) nach einem der Ansprüche 5 bis 6, wobei
der erste Schlüssel (12) eingerichtet ist, Daten, die von der ersten Infusionspumpe (2) versendet werden, mit einer eindeutigen und individuellen digitalen Signatur (16) zu versehen, um zu kennzeichnen, dass sie von der ersten Infusionspumpe (2) stammen, und
der zweite Schlüssel (14) eingerichtet ist, eine digitale Signatur (16) von Daten, die an die zweite Infusionspumpe (4) gesendet werden, zu prüfen und zu entscheiden, ob die digitale Signatur (16) von dem ersten Schlüssel (12) ist und die Daten somit von der ersten Infusionspumpe (2) stammen.

8. Infusionspumpensystem (20) nach Anspruch 7, welches eingerichtet ist, dass die zweite Infusionspumpe (4) Daten, für die der zweite Schlüssel (14) aufgrund der digitalen Signatur (16) entscheidet, dass sie von der ersten Infusionspumpe (2) stammen, annimmt und
Daten, für die der zweite Schlüssel (14) aufgrund der digitalen Signatur (16) entscheidet, dass sie nicht von der ersten Infusionspumpe (2) stammen, verwirft.

9. Infusionspumpensystem (20) nach einem der Ansprüche 5 bis 8, wobei
der zweite Schlüssel (14) eingerichtet ist, Daten so zu verschlüsseln, dass sie nur von dem ersten Schlüssel (12) entschlüsselt werden können, und
nur der erste Schlüssel (12) eingerichtet ist, Daten, die von dem zweiten Schlüssel (14) verschlüsselt sind, zu entschlüsseln.

10. Infusionspumpensystem (20) nach einem der Ansprüche 1 bis 9, wobei das Infusionspumpensystem (20) ferner einer Steuerungsinstanz (24) aufweist, welche dazu eingerichtet ist, dass sie verschlüsselte und/oder digital signierte Daten an zumindest eine aus der ersten und zweiten Infusionspumpe (2, 4) versendet und/oder von zumindest einer aus der ersten und zweiten Infusionspumpe (2, 4) verschlüsselte und/oder digital signierte Daten empfängt.

11. Verfahren zur sicheren Datenübertragung für ein Infusionspumpensystem (20), vorzugsweise nach einem der Ansprüche 1 bis 10, mit einer ersten Infusionspumpe (2) und einer zweiten Infusionspumpe (4), mit den Schritten:
- Koppeln der ersten und zweiten Infusionspumpe (2, 4);
- Erhalten von mit einer digitalen Signatur (16) signierten Daten für zumindest eine aus der ersten und zweiten Infusionspumpe (2, 4); und/oder
- Erhalten von mit einer Verschlüsselung (22) verschlüsselten Daten für zumindest eine aus der ersten und zweiten Infusionspumpe (2, 4).

12. Verfahren nach Anspruch 11, wobei der Schritt Koppeln der ersten und zweiten Infusionspumpe (2, 4) Folgendes aufweist:
- Erzeugen eines ersten Schlüssels (12) und eines zweiten Schlüssels (14) von der ersten Infusionspumpe (2);
- Erzeugen und Ausgeben einer Kopplungsinformation (18), vorzugsweise an einen Anwender (19) des Infusionspumpensystems (20), von der ersten Infusionspumpe (2);
- Übertragen der Kopplungsinformation (18) und des zweiten Schlüssels (14) an die zweite Infusionspumpe (4);
- Ausgeben der Kopplungsinformation (18) von der zweiten Infusionspumpe (4), vorzugsweise an den Anwender (19) des Infusionspumpensystems (20);
- Überprüfen der Kopplungsinformation (18), angezeigt von der ersten Infusionspumpe (2) und der zweiten Infusionspumpe (4), vorzugsweise durch den Anwender (19) des Infusionspumpensystems (20);
- Bei identischer Kopplungsinformation (18), Bestätigung dieser, vorzugsweise durch den Anwender (19) des Infusionspumpensystems (20), und Kopplung der ersten Infusionspumpe (2) und der zweiten Infusionspumpe (4) für das Erhalten von Daten mit einer digitalen Signatur (16) für zumindest eine aus der ersten und zweiten Infusionspumpe (2, 4) und/oder das Erhalten von verschlüsselten Daten für zumindest eine aus der ersten und zweiten Infusionspumpe (2, 4).

13. Verfahren nach einem der Ansprüche 11 bis 12, wobei das Verfahren vorgeordnet folgenden Schritt aufweist:
- Anfragen der zweiten Infusionspumpe (4) bei der ersten Infusionspumpe (2) für die Kopplung und/oder Anfragen der zweiten Infusionspumpe (4) bei einer Vielzahl von verfügbaren Infusionspumpen nach einer ersten Infusionspumpe (2) für Kopplung und Auswahl der ersten Infusionspumpe (2) aus der Vielzahl von verfügbaren Infusionspumpen.

14. Verfahren nach einem der Ansprüche 11 bis 13 mit folgenden weiteren Schritten:
- Versenden von Daten mit einer eindeutigen und individuellen digitalen Signatur (16), die von dem ersten Schlüssel (12) erstellt wird, von der ersten Infusionspumpe (2);
- Überprüfen der digitalen Signatur (16) von Daten mit dem zweiten Schlüssel (14) von der zweiten Infusionspumpe (4) und Entscheidung anhand der digitalen Signatur (16), ob die Daten von der ersten Infusionspumpe (2) stammen;
- Annehmen der Daten durch die zweite Infusionspumpe (4), wenn die Entscheidung ergibt, dass die Daten von der ersten Infusionspumpe (2) stammen; oder
- Verwerfen der Daten durch die zweite Infusionspumpe (4), wenn die Entscheidung ergibt, dass die Daten nicht von der ersten Infusionspumpe (2) stammen;
und/oder folgenden weiteren Schritten:
- Verschlüsseln von Daten mit dem zweiten Schlüssel (14) von der zweiten Infusionspumpe (4);
- Versenden der verschlüsselten Daten von der zweiten Infusionspumpe (4);
- Erhalten der verschlüsselten Daten von der ersten Infusionspumpe (2);
- Entschlüsseln der Daten mit dem ersten Schlüssel (12) von der ersten Infusionspumpe (2).

15. Computerlesbares Speichermedium (26), das Funktionen aufweist, die ein Infusionspumpensystem (20), bevorzugt nach einem der Ansprüche 1 bis 10, veranlassen, die Schritte des Verfahrens nach einem der Ansprüche 11 bis 14 auszuführen.
